# EUROPÄISCHE PATENTSCHRIFT

(11) **EP 1 878 380 B1**
(45) Veröffentlichungstag und Bekanntmachung des Hinweises auf die Patenterteilung: **10.11.2010**
(21) Anmeldenummer: 06014682.6
(22) Anmeldetag: 14.07.2006
(51) Int. Cl.: A61B 5/00, A61M 5/20, A61M 5/172

(54) **Injektionsgerät mit einer zweiten Gerätfunktion zum Gewinnen einer Körperflüssigkeitsprobe**
Injection device with a second function for sampling a probe
Appareil d'injection avec une deuxième fonction pour extraire un échantillon d'un liquide corporel

(43) Veröffentlichungstag der Anmeldung: 16.01.2008
(73) Patentinhaber: Roche Diagnostics GmbH, 68305 Mannheim (DE); F.Hoffmann-La Roche AG, 4070 Basel (CH)
(72) Erfinder: Jansen, Paul, 68163 Mannheim (DE); Rasch-Menges, Juergen, 68723 Schwetzingen (DE)
(74) Vertreter: Mommer, Niels

(56) Entgegenhaltungen:
- WO-A-93/02720
- FR-A- 2 040 830
- US-A- 4 850 973
- US-A- 5 536 249
- US-B1- 6 290 683

## Beschreibung

Die Erfindung betrifft ein Injektionsgerät zum Injizieren eines medizinischen Wirkstoffs, insbesondere Insulin, umfassend ein Gerätegehäuse, ein Aufnahmefach für eine Ampulle mit dem zu injizierenden Wirkstoff, eine Vorschubmechanik zum Bewegen einer in das Gerät eingesetzten Injektionsnadel in Einstichrichtung und eine Presseinrichtung zum Auspressen von Wirkstoff aus einer in das Aufnahmefach eingelegten Ampulle für einen Injektionsvorgang.

Derartige Injektionsgeräte sind bekannt und beispielsweise als so genannte "Insulin-pens" im Handel erhältlich. Sie werden insbesondere von insulinpflichtigen Diabetikern verwendet, um sich benötigte Insulindosen zu verabreichen. Ein derartiges Injektionsgerät ist beispielsweise aus der US 5536249 bekannt.

Aus der WO 93/02720 ist ein Injektionsgerät bekannt, das als zweite Gerätefunktion eine Probenentnahme ermöglicht, bei der durch die Injektionsnadel eine Probe eingesaugt wird. Ein Injektionsgerät mit dem durch Ansaugen eine Probe entnommen werden kann, ist auch in der FR 2040830 beschrieben.

Aus der US 4850973 ist ein Injektionsgerät bekannt, bei dem ebenfalls als zweite Gerätefunktion eine Probenentnahme ermöglicht ist. Damit die zweite Gerätefunktion genutzt werden kann, muss eine Injektionsspritze gegen einen Schlagbolzen und die Injektionsnadel gegen ein Lanzettenmodul ausgetauscht werden, das mit einem Bajohettverschluss an dem Gerät befestigt wird.

Die US 6290683 befasst sich mit dem Schutz vor einer unbeabsichtigten Verletzung durch Stechgeräte und beschreibt mehrere Ausführungsbeispiele von Stechgeräten, bei denen es sich entweder um Injektionsgeräte oder Lanzettengeräte für eine Probengewinnung handelt.

Für eine wirksame Therapie müssen insulinpflichtige Diabetiker mehrmals täglich ihren Glukosespiegel durch Messungen an einer geeigneten Körperflüssigkeit, üblicherweise Blut und/oder interstitieller Flüssigkeit, überprüfen. Hierzu wird mit einer geeigneten Stechhilfe eine Einstichwunde in einem Körperteil, üblicherweise einem Finger, erzeugt und aus der Einstichwunde gewonnenen Körperflüssigkeit mittels eines Teststreifens und einem Messgerät untersucht. In Abhängigkeit von dem dabei ermittelten Glukosespiegel wird anschließend die zu injizierende Insulindosis eingestellt und mittels eines Injektionsgeräts injiziert.

In das aus der US 5536249 bekannte Injektionsgerät ist ein Messgerät integriert, durch das mittels der dazugehörender Teststreifen der Blutglukosegehalt ermittelt werden kann.

Trotz im Laufe vieler Jahrzehnte erzielter Fortschritte bei der Entwicklung von Stechhilfen zum Erzeugen von Einstichwunden zur Gewinnung von Körperflüssigkeitsproben und Injektionsgeräten zu Injektion von Insulin bedeutet eine insulinpflichtige Diabeteserkrankung für die Betroffenen nach wie vor eine erhebliche Belastung und das für eine wirksame Therapie erforderliche mehrmals tägliche Messen und Injizieren von Insulin eine mühsame Last, die durch die Notwendigkeit erschwert wird, ständig ein Stechgerät, ein Injektionsgerät, Lanzetten für das Stechgerät, Injektionsnadeln für das Injektionsgerät, Insulinampullen und Teststreifen mitzuführen.

Aufgabe der Erfindung ist es deshalb, einen Weg aufzuzeigen, wie insulinpflichtigen Diabetikern eine optimale Therapie erleichtert werden kann.

Diese Aufgabe wird mit einem Injektionsgerät der eingangs genannten Art erfindungsgemäß dadurch gelöst, dass die Vorschubmechanik bei einer ersten Gerätefunktion für einen Injektionsvorgang ein erstes Bewegungsprofil einer eingesetzten Injektionsnadel bewirkt, bei dem an eine Vorschubbewegung eine Ruhephase anschließt, während der die eingesetzte Injektionsnadel für einen Injektionsvorgang relativ zu dem Gehäuse in Ruhe ist, und bei einer zweiten Gerätefunktion zum Gewinnen einer Körperflüssigkeitsprobe für diagnostische Zwecke ein zweites Bewegungsprofil einer eingesetzten Injektionsnadel bewirkt, bei dem an eine Einstichbewegung unmittelbar eine Rückführbewegung anschließt.

Ein erfindungsgemäßes Injektionsgerät vereint in sich die Funktionen einer herkömmlichen Stechhilfe, mit der eine Einstichwunde zum Gewinnen einer Körperflüssigkeitsprobe für diagnostische Zwecke erzeugt wird, und eines Injektionsgeräts zum Injizieren eines medizinischen Wirkstoffs, so dass ein insulinpflichtiger Diabetiker nur noch ein einziges Gerät mit sich führen muss. Dies bedeutet eine erhebliche Erhöhung des Benutzerkomforts und ermöglicht zudem eine Kostenersparnis bei der Behandlung insulinpflichtiger Diabetiker.

Um diese beiden Gerätefunktionen mit unterschiedlichen Anforderungen erfüllen zu können, ist erfindungsgemäß vorgesehen, dass die Injektionsnadel je nach Gerätefunktion mit unterschiedlichen Bewegungsprofilen bewegt wird. Bei der ersten Gerätefunktion, die zum Injizieren eines medizinischen Wirkstoffs dient, wird die Injektionsnadel mit einer Vorschubbewegung vorgeschoben und anschließend abgestoppt, so dass die Injektionsnadel für einen Injektionsvorgang relativ zu dem Gerätegehäuse in Ruhe ist. Beispielsweise kann bei der Vorschubbewegung die Spitze der Injektionsnadel aus einer Gehäuseöffnung herausgeschoben und der Einstich während der Ruhepause manuell vorgenommen werden.

Möglich ist es auch, dass die Injektionsnadel bereits während der Vorschubbewegung von einem Antrieb bis auf eine vorgegebene Einstichtiefe in die Haut eines Patienten eingestochen wird, also die Vorschubbewegung eine Einstichbewegung darstellt. Nach Erreichen der vorgegebenen Einstichtiefe wird in diesem Fall die Injektionsnadel abgestoppt und bleibt während der Ruhephase im Körper des Benutzers stecken. Während dieser Ruhephase erfolgt der eigentliche Injektionsvorgang, bei dem Wirkstoff aus der Ampulle ausgepresst und über die Injektionsnadel in den Körper des Patienten hineingepresst wird. Nach Abschluss des Injektionsvorgangs wird die Injektionsnadel aus der Einstichwunde herausgezogen, was manuell oder mittels eines Antriebs erfolgen kann. Die Einstichtiefe für diese erste Gerätefunktion beträgt typischerweise ca. 2 mm bis 12 mm, so dass die Injektionsnadel bei dem Injektionsvorgang in Unterhautfettgewebe hineinragt.

Wird der Einstich manuell im Anschluss an die Vorschubbewegung der ersten Gerätefunktion durchgeführt, ist für diese Vorschubbewegung kein Antrieb erforderlich, da für diese Vorschubbewegung keine großen Geschwindigkeiten erforderlich sind und die dafür benötigten Kräfte problemlos auch manuell aufgebracht werden können.

Bei der zweiten Gerätefunktion zum Gewinnen einer Körperflüssigkeitsprobe ist ein derart tiefer Einstich nicht erforderlich und würde nur unnötig Schmerzen verursachen. Neben einer möglichst geringen Einstichtiefe wird für einen schmerzarmen Einstich zur Gewinnung einer Körperflüssigkeitsprobe eine möglichst schnelle Einstich- und Rückführbewegung angestrebt. Bei der zweiten Gerätefunktion schließt deshalb an die Einstichbewegung unmittelbar eine schnelle Rückführbewegung an. Dies bedeutet, dass die Injektionsnadel während eines den Umkehrpunkt der Bewegung enthaltenden Bewegungsabschnitts, ständig einer von einem Antrieb erzeugten Kraft ausgesetzt ist, die eine Beschleunigung in Richtung der Rückzugsbewegung bewirkt. Der Begriff Beschleunigung ist hierbei in seinem physikalischen Sinn als zeitliche Ableitung der Geschwindigkeit zu verstehen. Zu Beginn des Bewegungsabschnitts bewirkt die von dem Antrieb bewirkte Beschleunigung zunächst ein Abbremsen der Vortriebsgeschwindigkeit der Einstichbewegung bis der Umkehrpunkt der Bewegung erreicht ist. Ab Erreichen des Umkehrpunkts, das heißt der eingestellten Einstichtiefe, bewirkt die Beschleunigung eine zunehmend schnellere Rückführbewegung. Der für die zweite Gerätefunktion erforderliche Antrieb ist Teil der Vorschubmechanik und kann auch für die erste Gerätefunktion genutzt werden, falls die Vorschubbewegung der ersten Gerätefunktion zum Erzeugen eines Einstichs genutzt wird.

Weitere Einzelheiten und Vorteile werden anhand von Ausführungsbeispielen unter Bezugnahme auf die beigefügten Zeichnungen erläutert. Gleiche und einander entsprechende Bauteile sind darin mit übereinstimmenden Bezugszahlen gekennzeichnet. Die im Folgenden erläuterten Merkmale können einzeln oder in Kombination zum Gegenstand von Ansprüchen gemacht werden. Es zeigen:
- Fig. 1: ein Ausführungsbeispiel eines erfindungsgemäßen Injektionsgerät in einer Seitenansicht;
- Fig. 2: das in Figur 1 dargestellte Ausführungsbeispiel mit aufgeschnittenem Gehäuse;
- Fig. 3: eine vereinfachte Darstellung des in Figur 1 dargestellten Ausführungsbeispiels in einem Querschnitt;
- Fig. 4: ein weiteres Ausführungsbeispiel eines erfindungsgemäßen Injektionsgeräts in einer schematischen Querschnittsansicht;
- Fig. 5: eine Detailansicht der Stirnkappe der in den Figuren 1 und 4 dargestellten Ausführungsbeispiele;
- Fig. 6: ein weiteres Ausführungsbeispiel einer Stirnkappe; und
- Fig. 7: ein weiteres Ausführungsbeispiel einer Stirnkappe.

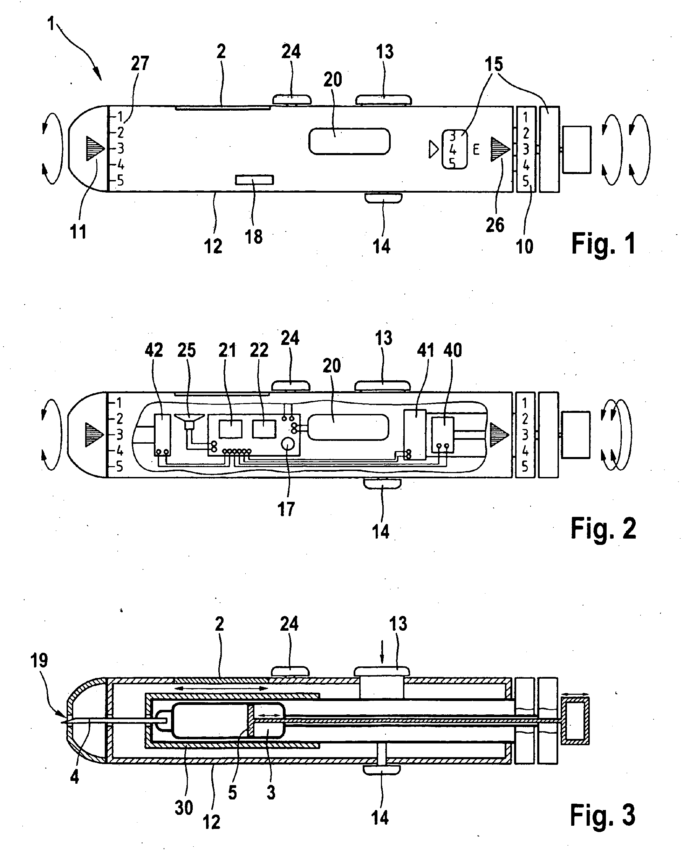

Das in den Figuren 1 bis 3 dargestellte Ausführungsbeispiel eines Injektionsgeräts 1 zum Injizieren von Insulin umfasst in herkömmlicher Weise ein Aufnahmefach 2 für eine Insulinampulle 3, eine Vorschubmechanik mit einem Antrieb zum Bewegen einer in das Gerät eingesetzten, austauschbaren Injektionsnadel 4 für eine Einstichbewegung zum Erzeugen einer Einstichwunde in der Haut eines Benutzers und eine Presseinrichtung 5 zum Auspressen von Insulin aus einer in das Aufnahmefach eingelegten Insulinampulle 3 für ein Injektionsvorgang. Das dargestellte Injektionsgerät 1 zeichnet sich dadurch aus, dass es zusätzlich zu einer ersten Gerätefunktion, die dem Injizieren von Insulin dient, eine zweite Gerätefunktion hat, die dem Erzeugen einer Einstichwunde zum Gewinnen einer Körperflüssigkeitsprobe für diagnostische Zwecke dient. Bei beiden Gerätefunktionen bewirkt der Antrieb eine Einstichbewegung einer in das Gerät eingesetzten Injektionsnadel 4 zum Erzeugen einer Einstichwunde in der Haut eines Benutzers.

Bei der ersten Gerätefunktion bewirkt der Antrieb ein erstes Bewegungsprofil der eingesetzten Injektionsnadel 4, bei dem an eine Vorschubbewegung eine Ruhephase anschließt, während der die eingesetzte Injektionsnadel für einen Injektionsvorgang relativ zu Gerätegehäuse 12 in Ruhe ist. Bevorzugt wird mit der Vorschubbewegung eine Spitze einer in das Gerät eingesetzten Injektionsnadel 4 aus einer Gehäuseöffnung 19 herausgeschoben. Während der Ruhepause kann mit der vorgeschobenen Injektionsnadel manuell in Körpergewebe eingestochen werden. Möglich ist es aber auch, dass der Einstich bereits während der Vorschubbewegung automatisch erfolgt und die Injektionsnadel 4 während der anschließenden Ruhepause in der erzeugten Einstichwunde verbleibt. Die Zeitdauer der Ruhephase hängt unter anderem von der zu injizierenden Insulinmenge ab, und beträgt im Regelfall einige Sekundenbruchteile oder wenige Sekunden. Die Einstichtiefe, mit der die Injektionsnadel bei der ersten Gerätefunktion in die Haut eines Patienten eingestochen wird, beträgt typischerweise 2 mm bis 12 mm, so dass die Injektionsnadel bei dem Injektionsvorgang in Unterhautfettgewebe hineinragt.

Bei der zweiten Gerätefunktion wird mit der Injektionsnadel eine Einstichwunde zum Gewinnen einer Körperflüssigkeitsprobe für diagnostische Zwecke erzeugt. Bei der Körperflüssigkeit, die durch eine mit der zweiten Gerätefunktion erzeugten Einstichwunde gewonnen wird, handelt es sich üblicherweise um Blut und/oder interstitieller Flüssigkeit. Anhand einer derartigen Körperflüssigkeitsprobe kann der Glukosegehalt bestimmt und damit die benötigte Insulindosis ermittelt werden. Wesentlich für die zweite Gerätefunktion ist, dass die Einstichwunde zum Gewinnen der Körperflüssigkeitsprobe für diagnostische Zwecke möglichst schmerzarm, im Idealfall schmerzfrei, erfolgt. Dies macht eine möglichst geringe Einstichtiefe und eine möglichst schnelle Einstich- und Rückführbewegung erforderlich. Ein Einstich zur Gewinnung einer Körperflüssigkeitsprobe für diagnostische Zwecke sollte nur so tief sein, wie es zum Gewinnen der Körperflüssigkeitsprobe unbedingt erforderlich ist. Um diese Anforderungen zu erfüllen, bewirkt der Antrieb bei der zweiten Gerätefunktion ein zweites Bewegungsprofil der eingesetzten Injektionsnadel 4, bei dem an eine Einstichbewegung unmittelbar eine Rückführbewegung anschließt.

Aus dem gesagten geht hervor, dass die Einstichtiefe der Injektionsnadel für einen komfortablen Gebrauch des Injektionsgeräts 1 von großer Bedeutung ist. Das Injektionsgerät umfasst deshalb eine Einstelleinrichtung 10, 11 zum Einstellen einer ersten Einstichtiefe für das erste Bewegungsprofil für einen Injektionsvorgang und einer zweiten Einstichtiefe für das zweite Bewegungsprofil zum Gewinnen einer Körperflüssigkeitsprobe für diagnostische Zwecke. Zum Erfassen der eingestellten Einstichtiefe umfasst das Injektionsgerät 1 einen oder mehrere in Figur 2 dargestellte Sensoren 41, 42. Bei dem dargestellten Ausführungsbeispiel umfasst die Einstelleinrichtung ein erstes manuell betätigbares Einstellelement 10 durch dessen Betätigung ein Benutzer die Einstichtiefe des ersten Bewegungsprofils einstellen kann, sowie ein zweites manuell betätigbares Einstellelement 11, durch dessen Betätigung ein Benutzer die Einstichtiefe des zweiten Bewegungsprofils einstellen kann. Bei dem ersten Einstellelement 10 handelt es sich um ein Stellrad, durch dessen Drehen die erste Einstichtiefe für die Injektion von Insulin eingestellt werden kann. Bei dem zweiten Einstellelement 11 handelt es sich um eine aufgeschraubte Stirnkappe, die gegenüber einem zylindrischen Gerätegehäuse 12 drehbar ist, wobei sich durch Drehen der Abstand der Stirnkappe 11 und damit einer Geräteöffnung 19 zu dem zylindrischem Gerätegehäuse ändert.

Zum Erzeugen einer Einstichwunde wird das Injektionsgerät 1 mit der Stirnkappe an ein Körperteil angepresst. Bei einem Einstichvorgang tritt die Injektionsnadel 4 durch die Geräteöffnung 19 heraus und in das angepresste Körperteil hinein. Möglich ist es selbstverständlich auch, die Geräteöffnung 19 so auszubilden, dass sich Gewebe eines angepressten Körperteils in die Stirnkappe hineinwölbt, so dass eine Einstichwunde auch ohne austreten der Injektionsnadel 4 aus dem Gerätinneren erzeugt werden kann.

Prinzipiell ist es nicht erforderlich, dass die Einstelleinrichtung zwei separate manuell betätigbare Einstellelemente 10, 11 aufweist. Es hat sich jedoch gezeigt, dass die Bedienbarkeit eines Injektionsgeräts 1, bei dem ein erstes Einstellelement 10 dem Einstellen einer Einstichtiefe der ersten Gerätefunktion und ein zweites Einstellelement 11 dem Einstellen der Einstichtiefe einer zweiten Gerätefunktion dient, wesentlich besser ist, da für Benutzer intuitiv klar ist, welche der beiden Einstichtiefen durch Betätigen des entsprechenden Einstellelements geändert wird und somit die Gefahr von Fehlbedienungen reduziert ist.

Zu der Einstelleinrichtung 10, 11 gehört bei dem dargestelltem Ausführungsbeispiel eine Anzeigeeinrichtung 26, 27 zum Anzeigen der eingestellten Einstichtiefe. Dabei ist es günstig, wenn mit der Anzeigeeinrichtung gleichzeitig eine eingestellte erste Einstichtiefe des ersten Bewegungsprofils und eine eingestellte zweite Einstichtiefe des zweiten Bewegungsprofils angezeigt werden kann. Bei dem dargestellten Ausführungsbeispiel ist dies dadurch gegeben, dass die drehbaren Einstellelemente 10, 11 mit Markierungen versehen sind, die mit drehfest am Gehäuse 12 angebrachten Markierungen 26, 27 zusammenwirken und so das Ablesen der eingestellten Einstichtiefen ermöglichen. Bei dem Einstellelement 10 ist das Stellrad mit Ziffern versehen, die mit der an dem Gehäuse 12 angeordneten Markierung 26 in Form eines Dreiecks oder Pfeils zusammenwirken. Ein Benutzer kann durch einen Blick erkennen, auf welche der Ziffern das an dem Gehäuse 12 angeordnete Dreieck 26 zeigt und daraus auf die eingestellte Einstichtiefe des ersten Bewegungsprofils schließen. Bei dem zweiten Einstellelement ist ein entsprechendes Dreieck gegenüber dem Gehäuse 12 drehbar, während eine Ziffernfolge auf dem Gehäuse angebracht ist und die Anzeigeeinrichtung 27 bildet. Auch bei dem Einstellelement 11 kann ein Benutzer durch einen Blick erkennen, auf welche der Ziffern das Dreieck zeigt und so auf die eingestellte Einstichtiefe des Bewegungsprofils zwei schließen.

Das dargestellte Injektionsgerät 1 hat ferner eine Aktivierungseinrichtung, mit der wahlweise eine Bewegung der Injektionsnadel 4 mit dem ersten Bewegungsprofil oder mit dem zweiten Bewegungsprofil ausgelöst werden kann. Bei dem dargestellten Ausführungsbeispiel umfasst die Aktivierungseinrichtung ein erstes manuell betätigbares Auslöseelement 13, durch dessen Betätigung ein Benutzer eine Bewegung der Injektionsnadel mit dem ersten Bewegungsprofil auslösen kann, und ein zweites manuell betätigbares Auslöseelement 14, durch dessen Betätigung ein Benutzer eine Bewegung der Injektionsnadel 4 mit dem zweiten Bewegungsprofil auslösen kann. Bei dem dargestellten Ausführungsbeispiel sind die Auslöseelemente 13, 14 als Tasten ausgebildet. Die Verwendung getrennter Auslöseelemente 13, 14 für die beiden Gerätefunktionen erleichtert es einem Benutzer, die jeweils gewünschte Gerätefunktion zu aktivieren. Selbstverständlich ist jedoch prinzipiell ein einziges manuell betätigbares Auslöseelement ausreichend, sofern durch andere Maßnahme ausgewählt werden kann, welche der beiden Gerätefunktionen durch Betätigen des Auslöseelements aktiviert werden.

Das dargestellte Injektionsgerät 1 hat eine Dosiereinrichtung 15 zum Einstellen der bei einem Injektionsvorgang aus einer eingelegten Insulinampulle auszupressenden Wirkstoffmenge. Die Dosiereinrichtung 15 hat ein manuell betätigbares Stellrad, dessen Position und damit die eingestellte Insulinmenge durch ein Sichtfensters des Gehäuses 12 angezeigt wird. Der Füllzustand einer eingesetzten Insulinampulle kann visuell überprüft werden, da das Gehäuse 12 im Bereich des Insulinfachs 2 transparent ist, so dass ein Benutzer leicht sehen kann, wie viel Insulin in einer eingesetzten Ampulle 3 noch vorhanden ist.

Das dargestellte Injektionsgerät 1 hat eine integrierte Messeinheit 17 zum Untersuchen einer Körperflüssigkeitsprobe, die mittels einer Einstichwunde der Injektionsnadel 4 nach dem zweiten Bewegungsprofil gewonnen werden kann. Bei dem dargestellten Ausführungsbeispiel ist die Messeinheit 17 dafür eingerichtet, den Glukosegehalt einer Körperflüssigkeitsprobe mittels handelsüblicher Teststreifen zu bestimmen.

In dem Gehäuse 12 des Injektionsgeräts 1 ist ein Schlitz 18 angeordnet, in den ein Testelement, beispielsweise in Form eines handelsüblichen Teststreifens, das mit einer Körperflüssigkeitsprobe benetzt wurde, zur Messung der Glukosekonzentration eingeschoben werden kann. Nach Benetzung des Testelements mit Körperflüssigkeit ergibt sich eine Verfärbung des Testelements, deren Intensität von der Glukosekonzentration der Körperflüssigkeitsprobe abhängt. Der Grad der Verfärbung kann photometrisch mittels der Messeinheit 17 ermittelt werden. Als Alternative zu photometrischen Messeinheiten sind elektrochemische Messeinheiten bekannt.

Die Messeinheit 17 ist mit einer zentralen Auswerte- und Steuerungseinheit 21 verbunden, die an eine Anzeigeeinrichtung 20 in Form eines Flüssigkristalldisplays angeschlossen ist, mit dem ein ermittelter Glukosegehalt angezeigt werden kann. Die Auswerte- und Steuerungseinheit 21 ist bevorzugt als ASIC (applification specific integrated circuit) ausgebildet, der zusammen mit einem Speicher 22 auf einer Platine angeordnet ist.

Die Messeinheit 17 wird mittels des Bedienelements 24 betätigt. Die Anzeigeeinrichtung 20, bei der es sich um ein Flüssigkristalldisplay mit Segmentanzeige handelt, kann zusätzlich zum Anzeigen von Messergebnissen auch zum Anzeigen von Geräteinformationen verwendet werden. Die Auswerte- und Steuereinheit 21 ist mit einer Schnittstelle 25 verbunden, über die mit externen Systemen, beispielsweise einem PC oder einer Insulinpumpe, Daten ausgetauscht werden können. Die Schnittstelle 25 kann als kabelgebundete serielle Schnittstelle oder Infrarotschnittstelle ausgebildet sein. Möglich ist auch eine RF-Schnittstelle sowie aufwendigere Schnittstellen gemäß Bluetooth oder WLAN. Damit das Gerät netzunabhängig betrieben werden kann, enthält es eine Energiequelle, beispielsweise in Form von Batterien oder Solarzellen.

Über die Schnittstelle 25 können beispielsweise Daten über die bei einem Injektionsvorgang injizierte Insulinmenge ausgegeben werden. Das Injektionsgerät 1 umfasst hierfür einen in Figur 2 dargestellten Sensor 40 zum Erfassen der Injektionsmenge. Der Sensor 40 ist mit der Auswerte- und Steuereinheit 21 verbunden und setzt bei dem dargestellten Ausführungsbeispiel eine mechanische Bewegung der Dosiereinrichtung 15 in elektrische Signale um. Die Dosiereinrichtung 15 umfasst ein manuell betätigbares Stellrad, dessen Drehbewegung mit dem Sensor 40 gemessen wird, so dass die jeweilige Geräteeinstellung hinsichtlich der zu injizierenden Insulinmenge durch Auswertung der Signale 40 ermittelt werden kann.

Wie bereits erwähnt, umfasst das Injektionsgerät 1 zum Erfassen der eingestellten Einstichtiefen Sensoren 41, 42, die ebenfalls an die Auswerte- und Steuereinheit 21 angeschlossen sind. Die Sensoren 41, 42 messen eine mechanische Bewegung der Einstelleinrichtung 10, 11 und setzten diese im Betrieb in elektrische Signale um, aus denen sich die eingestellte Einstichtiefe ermitteln lässt. Die mittels der Sensoren 40, 41, 42 erfassten mechanisch vorgenommenen Geräteeinstellungen, die bei dem dargestellten Ausführungsbeispiel durch Drehbewegungen, beispielsweise der Einstellelemente 10, 11 oder des Stellrads der Dosiereinrichtung 15, vorgenommen werden, werden von der Auswerte- und Steuereinheit 21 gespeichert und können über die Schnittstelle 25 übertragen werden.

Bei dem Antrieb des in Figur 1 dargestellten Injektionsgeräts handelt es sich um einen Federantrieb. Federantriebe für Injektionsgeräte und Stechgeräte, mit denen Einstichwunden zur Gewinnung von Körperflüssigkeitsproben für diagnostische Zwecke erzeugt werden, sind dem Fachmann geläufig und beispielsweise aus der EP 0565970 B1 bekannt, die diesbezüglich durch Bezugnahme zum Gegenstand der vorliegenden Anmeldung gemacht wird. Auf eine Abbildung und nähere Beschreibung von konstruktiven Einzelheiten des Federantriebs wird deshalb verzichtet.

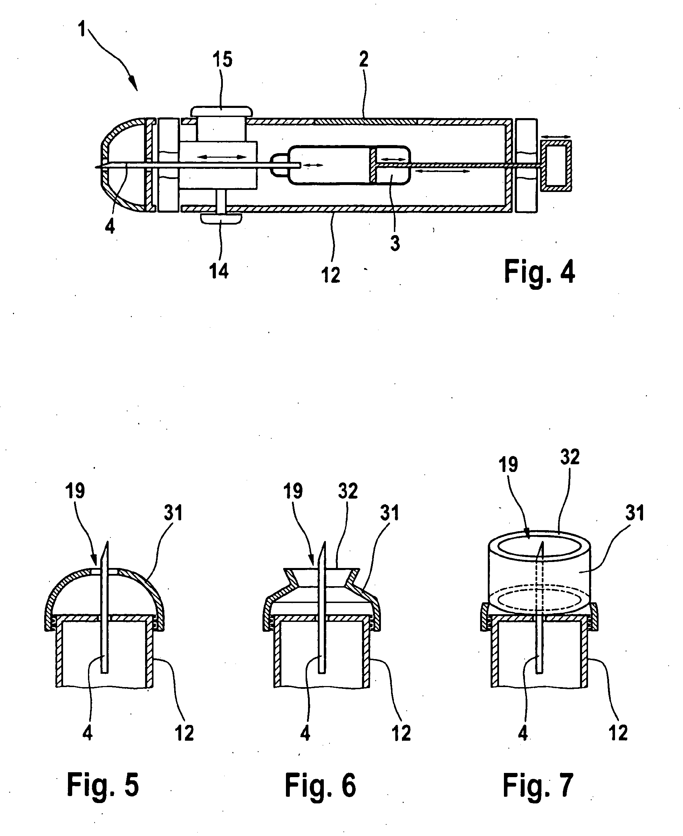

Bei dem dargestellten Ausführungsbeispiel wird von dem Antrieb bei einer Einstichbewegung die Injektionsnadel 4 zusammen mit der Insulinampulle 3 bewegt. Die Insulinampulle wird dabei von einem in Einstichrichtung beweglichen Schlitten 30 getragen, der von dem Antrieb bewegt wird. Zur Reibungsminimierung ist der Schlitten auf Führungselementen, bevorzugt Schienen, verschiebbar gelagert. Bei dem in Figur 3 dargestellten Ausführungsbeispiel wird der Schlitten 30 mit der Insulinampulle 3 und der Injektionsnadel 4 sowohl bei der ersten Gerätefunktion als auch bei der zweiten Gerätefunktion in Einstichrichtung verfahren. Für eine Einstichbewegung genügt es jedoch prinzipiell die Einstichnadel 4 zu bewegen, wobei die Insulinampulle 3 gegenüber dem Gehäuse 12 unbewegt bleiben kann. Ein Ausführungsbeispiel eines entsprechenden Injektionsgeräts ist in Figur 4 dargestellt. Besonders günstig ist es, diese beiden Antriebsmöglichkeiten miteinander zu kombinieren, das heißt bei einer der beiden Gerätefunktionen die Einstichnadel 4 zusammen mit der Insulinampulle 3 zu bewegen und bei der anderen Gerätefunktion die Injektionsnadel 4 relativ zu der Insulinampulle, die gegenüber dem Gehäuse ortsfest bleiben kann, zu bewegen. Auf diese Weise lassen sich wegen der getrennten Bewegungsmechanismen die beiden Einstichtiefen für das erste und das zweite Bewegungsprofil besonders einfach unabhängig voneinander einstellen und die beiden Bewegungsprofile unabhängig voneinander aktivieren. Dabei ist es besonders günstig bei dem zweiten Bewegungsprofil die Injektionsnadel 4 gemeinsam mit der Ampulle 3 zu bewegen, da dies eine besonders schnelle Einstich- und Rückführbewegung ermöglicht, und bei dem ersten Bewegungsprofil die Injektionsnadel 4 relativ zu der Ampulle 3 zu bewegen.

Das Injektionsgerät 1 wird zum Erzeugen einer Einstichwunde mit seiner Stirnseite an ein Körperteil eines Benutzers angepresst. An seiner Stirnseite hat das Injektionsgerät 1 eine abnehmbare Stirnkappe 31, die mit einer Öffnung 19 versehen ist, durch welche die Injektionsnadel 4 bei einem Einstich austreten kann. In den Figuren 5 bis 7 sind verschiedene Ausführungsbeispiele geeigneter Stirnkappen 31 dargestellt. Insbesondere bei dem in Figur 6 dargestellten Ausführungsbeispiel ist die Öffnung 19, so groß, dass sich eine dagegen gepresste Fingerbeere in die Öffnung 19 hineinwölbt und folglich ein Austreten der Injektionsnadel 4 aus dem Inneren zum Erzeugen einer Einstichwunde nicht unbedingt erforderlich ist. Um selbst bei minimaler Einstichtiefe mit der zweiten Gerätefunktion eine für diagnostische Zwecke ausreichende Körperflüssigkeitsprobe zu gewinnen, ist es günstig, wenn die Stirnkappe 31 an der Geräteöffnung 19 eine trichterförmige Andruckfläche 32 aufweist, die sich an eine angepresste Fingerbeere anschmiegt und deren Durchblutung fördert. Auf diese Weise lässt sich durch die verbesserte Durchblutung leichter eine ausreichend große Körperflüssigkeitsprobe gewinnen.

Die Stirnkappe 31 kann für die erste Gerätefunktion, mit der Insulin injiziert wird, abgenommen werden, um die Blutung einer zur Insulininjektion erzeugten Einstichwunde zu minimieren. Die Stirnkappen 31 sind bevorzugt durch Aufschrauben an dem Gerätegehäuse 12 befestigt. Mittels eines geeigneten Sensors kann festgestellt werden, ob an dem Gehäuse 12 eine Stirnkappe 31 angebracht ist. Die Auswerte- und Steuereinheit 21 kann anhand des Sensorergebnisses jeweils die erste oder die zweite Gerätefunktion blockieren, so dass bei aufgesetzter Stirnkappe 31 nur die zweite Gerätefunktion und bei fehlender Stirnkappe 31 nur die erste Gerätefunktion aktiviert werden kann.

## Patentansprüche

1. Injektionsgerät zum Injizieren eines medizinischen Wirkstoffs, insbesondere Insulin, umfassend:
ein Gehäuse (12),
ein Aufnahmefach (2) für eine Ampulle (3) mit dem zu injizierenden Wirkstoff,
eine Vorschubmechanik zum Bewegen einer in das Gerät (1) eingesetzten Injektionsnadel (4) in einer Einstichrichtung und
eine Presseinrichtung (5) zum Auspressen von Wirkstoff aus einer in das Aufnahmefach (2) eingelegten Ampulle (3) für einen Injektionsvorgang,
die Vorschubmechanik
- bei einer ersten Gerätefunktion für einen Injektionsvorgang ein erstes Bewegungsprofil einer eingesetzten Injektionsnadel (4) bewirkt, bei dem an eine Vorschubbewegung eine Ruhephase anschließt, während der die eingesetzte Injektionsnadel (4) für einen Injektionsvorgang relativ zu dem Gehäuse in Ruhe ist, und
- bei einer zweiten Gerätefunktion zum Gewinnen einer Körperflüssigkeitsprobe für diagnostische Zwecke ein zweites Bewegungsprofil einer eingesetzten Injektionsnadel (4) bewirkt, bei dem an eine Einstichbewegung unmittelbar eine Rückführbewegung anschließt **dadurch gekennzeichnet, dass**, die Vorschubmechanik bei einer zweiten Gerätefunktion die Injektionsnadel während eines den Umkehrpunkt der Bewegung enthaltenden Bewegungsabschnitts ständig einer von einem Antrieb erzeugten Kraft aussetzt.

2. Injektionsgerät nach Anspruch 1, **gekennzeichnet durch** eine Aktivierungseinrichtung, mit der wahlweise eine Bewegung der Injektionsnadel (4) mit dem ersten oder dem zweiten Bewegungsprofil ausgelöst werden kann.

3. Injektionsgerät nach Anspruch 2, **dadurch gekennzeichnet, dass** die Aktivierungseinrichtung ein erstes manuell betätigbares Auslöselement (13), durch dessen Betätigung ein Benutzer eine Bewegung der Injektionsnadel (4) mit dem ersten Bewegungsprofil auslösen kann, und ein zweites manuell betätigbares Auslöseelement (14), durch dessen Betätigung ein Benutzer eine Bewegung der Injektionsnadel (4) mit dem zweiten Bewegungsprofil auslösen kann, umfasst.

4. Injektionsgerät nach einem der vorhergehenden Ansprüche, **gekennzeichnet durch** eine Einstelleinrichtung (10, 11) zum Einstellen einer ersten Einstichtiefe für das erste Bewegungsprofil für einen Injektionsvorgang und einer zweiten Einstichtiefe für das zweite Bewegungsprofil zum Gewinnen einer Körperflüssigkeitsprobe für diagnostische Zwecke.

5. Injektionsgerät nach Anspruch 4, **gekennzeichnet durch** eine Anzeigeeinrichtung (26, 27) zum gleichzeitigen Anzeigen einer eingestellten ersten und einer eingestellten zweiten Einstichtiefe.

6. Injektionsgerät nach Anspruch 4 oder 5, **dadurch gekennzeichnet, dass** die Einstelleinrichtung ein erstes manuell betätigbares Einstellelement (13) zum Einstellen der ersten Einstichtiefe des ersten Bewegungsprofils und ein zweites manuell betätigbares Einstellelement (14) zum Einstellen der zweiten Einstichtiefe für das zweite Bewegungsprofil umfasst.

7. Injektionsgerät nach einem der vorhergehenden Ansprüche, **gekennzeichnet durch** eine Messeinheit (17) zum Untersuchen einer mittels einer Einstichbewegung der Injektionsnadel (4) nach dem zweiten Bewegungsprofil gewonnenen Körperflüssigkeitsprobe.

8. Injektionsgerät nach einem der vorhergehenden Ansprüche, **gekennzeichnet durch** eine abnehmbare Stirnkappe (31), mit der das Injektionsgerät (1) bei der zweiten Gerätefunktion bestimmungsgemäß an ein Körperteil zum Gewinnen einer Körperflüssigkeitsprobe angedrückt wird.

9. Injektionsgerät nach einem der vorhergehenden Ansprüche, **dadurch gekennzeichnet, dass** die Vorschubmechanik bei mindestens einem der Bewegungsprofile eine eingesetzte Injektionsnadel (4) relativ zu einer eingesetzten Ampulle (3) bewegt.

10. Injektionsgerät nach einem der vorhergehenden Ansprüche, **dadurch gekennzeichnet, dass** die Vorschubmechanik bei mindestens einem der beiden Bewegungsprofile eine eingesetzte Injektionsnadel (4) gemeinsam mit einer eingesetzten Ampulle (3) bewegt.

11. Injektionsgerät nach einem der vorhergehenden Ansprüche, **dadurch gekennzeichnet, dass** der Antrieb bei einem der beiden Bewegungsprofile eine eingesetzte Injektionsnadel (4) relativ zu einer eingesetzten Ampulle (3) bewegt und bei dem anderen der beiden Bewegungsprofile eine eingesetzte Injektionsnadel (4) gemeinsam mit einer eingesetzten Ampulle (3) bewegt.

12. Injektionsgerät nach einem der vorhergehenden Ansprüche, **gekennzeichnet durch** einen Sensor (40, 41, 42) zum Erfassen einer mechanisch vorgenommenen Geräteeinstellung, insbesondere hinsichtlich der eingestellten Einstichtiefe oder der zu injizierenden Insulinmenge.

## Claims

1. Injection device for the injection of a medical agent, in particular insulin, comprising:
a housing (12),
a reception compartment (2) for a vial (3) containing the agent to be injected,
advancement mechanics for moving an injection needle (4) that is inserted in the device (1) in a direction of puncturing, and
a pressing facility (5) for pressing agent from a vial (3) that is inserted in the reception compartment (2) for the purpose of an injection process,
the advancement mechanics,
- effects, in a first device function for an injection process, a first motion profile of an inserted injection needle (4), in which a resting phase follows after an advancement motion, during which resting phase the inserted injection needle (4) for an injection process is at rest with respect to the device housing, and
- effects, in a second device function for obtaining a body fluid sample for diagnostic purposes, a second motion profile of an inserted injection needle (4), in which a returning motion follows immediately after a puncturing motion, **characterized in that** the advancement mechanics in the second device function during a motion part containing the reversal point continuously exerts a force on the needle.

2. Injection device according to claim 1, **characterized by** an activation facility that can be used to optionally initiate a motion of the injection needle (4) with the first or the second motion profile.

3. Injection device according to claim 2, **characterized in that** the activation facility comprises a first manually-actuated trigger element (13), the actuation of which allows a user to trigger a motion of the injection needle (4) with the first motion profile, and a second manually-actuated trigger element (14), the actuation of which allows a user to trigger a motion of the injection needle (4) with the second motion profile.

4. Injection device according to any one of the preceding claims, **characterized by** a setting facility (10, 11) for setting a first puncturing depth for the first motion profile for an injection process and a second puncturing depth for the second motion profile for obtaining a body fluid sample for diagnostic purposes.

5. Injection device according to claim 4, **characterized by** a display facility (26, 27) for the simultaneous display of a set first and a set second puncturing depth.

6. Injection device according to claim 4 or 5, **characterized in that** the activation facility comprises a first manually-actuated setting element (13) for setting the first puncturing depth of the first motion profile and a second manually-actuated setting element (14) for setting the second puncturing depth of the second motion profile.

7. Injection device according to any one of the preceding claims, **characterized by** a measuring unit (17) for assaying a body fluid sample that is obtained by means of a puncturing motion of the injection needle (4) according to the second motion profile.

8. Injection device according to any one of the preceding claims, **characterized by** a removable front cap (31) for being pressed together with the injection device (1) to a body part for obtaining a body fluid sample in the second device function.

9. Injection device according to any one of the preceding claims, **characterized in that** the advancement mechanics moves an inserted injection needle (4) with respect to an inserted vial (3) in at least one of the motion profiles.

10. Injection device according to any one of the preceding claims, **characterized in that** the advancement mechanics moves an inserted injection needle (4) jointly with an inserted vial (3) in at least one of the two motion profiles.

11. Injection device according to any one of the preceding claims, **characterized in that** the advancement mechanics moves an inserted injection needle (4) with respect to an inserted vial (3) in one of the two motion profiles and moves an inserted injection needle (4) jointly with an inserted vial (3) in the other of the two motion profiles.

12. Injection device according to any one of the preceding claims, **characterized by** a sensor (40, 41, 42) for detecting a device setting that has been set mechanically, in particular regarding the set puncturing depth or the set quantity of insulin to be injected.

## Revendications

1. Appareil d'injection pour l'injection d'une substance active médicale, en particulier d'insuline, comprenant :
un logement (12) ;
un compartiment de réception (2) pour une ampoule (3) comprenant la substance active à injecter ;
un mécanisme d'avance pour déplacer une aiguille d'injection (4) insérée dans l'appareil (1) dans une direction de piqûre ; et
un dispositif de compression (5) pour extraire la substance active par compression hors d'une ampoule (3) insérée dans le compartiment de réception (2) pour un processus d'injection ;
le mécanisme d'avance
- dans une première fonction de l'appareil pour un processus d'injection mettant en oeuvre un premier profil de déplacement d'une aiguille d'injection insérée (4), dans lequel une phase de repos fait directement suite à un mouvement d'avance, phase au cours de laquelle l'aiguille d'injection insérée (4) pour un processus d'injection reste au repos par rapport au logement, et
- dans une deuxième fonction de l'appareil, pour obtenir un échantillon de fluide organique à des fins de diagnostic, met en oeuvre un deuxième profil de mouvement d'une aiguille d'injection insérée (4) dans lequel un mouvement de retour fait directement suite à un mouvement de piqûre, **caractérisé en ce que** le mécanisme d'avance, dans une deuxième fonction de l'appareil, expose l'aiguille d'injection, au cours d'un tronçon de mouvement comportant le point d'inversion du mouvement, constamment à une force générée par un entraînement.

2. Appareil d'injection selon la revendication 1, **caractérisé par** un dispositif d'activation avec lequel on peut déclencher, selon ce que l'on souhaite, un mouvement de l'aiguille d'injection (4) avec le premier ou avec le deuxième profil de déplacement.

3. Appareil d'injection selon la revendication 2, **caractérisé en ce que** le dispositif d'activation comprend un premier élément de déclenchement (13) qui peut être actionné manuellement, par l'actionnement duquel un utilisateur peut déclencher un mouvement de l'aiguille d'injection (4) avec le premier profil de déplacement et un deuxième élément de déclenchement (14) qui peut être actionné manuellement, par l'actionnement duquel un utilisateur peut déclencher un mouvement de l'aiguille d'injection (4) avec le deuxième profil de déplacement.

4. Appareil d'injection selon l'une quelconque des revendications précédentes, **caractérisé par** un dispositif de réglage (10, 11) pour le réglage d'une première profondeur de piqûre pour le premier profil de déplacement pour un processus d'injection et une deuxième profondeur de piqûre pour le deuxième profil de déplacement destiné à l'obtention d'un échantillon de fluide organique à des fins de diagnostic.

5. Appareil d'injection selon la revendication 4, **caractérisé par** un dispositif d'affichage (26, 27) pour l'affichage simultané d'une première profondeur de piqûre réglée et d'une deuxième profondeur de piqûre réglée.

6. Appareil d'injection selon la revendication 4 ou 5, **caractérisé en ce que** le dispositif de réglage comprend un premier élément de réglage (13) qui peut être actionné à la main pour le réglage de la première profondeur de piqûre du premier profil de déplacement et un deuxième élément de réglage (14) qui peut être actionné à la main pour le réglage de la deuxième profondeur de piqûre destiné au deuxième profil de déplacement.

7. Appareil d'injection selon l'une quelconque des revendications précédentes, **caractérisé par** une unité de mesure (17) pour l'analyse d'un échantillon de fluide organique récupéré au moyen d'un mouvement de piqûre de l'aiguille d'injection (4) conformément au deuxième profil de déplacement.

8. Appareil d'injection selon l'une quelconque des revendications précédentes, **caractérisé par** un capuchon frontal amovible (31) avec lequel l'appareil d'injection (1) peut être comprimé, dans la deuxième fonction de l'appareil, comme il se doit, contre une partie du corps pour récupérer un échantillon de fluide organique.

9. Appareil d'injection selon l'une quelconque des revendications précédentes, **caractérisé en ce que** le mécanisme d'avance, dans au moins un des deux profils de déplacement, déplace une aiguille d'injection insérée (4) par rapport à une ampoule insérée (3).

10. Appareil d'injection selon l'une quelconque des revendications précédentes, **caractérisé en ce que** le mécanisme d'avance, dans au moins un des deux profils de déplacement, déplace une aiguille d'injection insérée (4) conjointement avec une ampoule insérée (3).

11. Appareil d'injection selon l'une quelconque des revendications précédentes, **caractérisé en ce que** l'entraînement, dans un des deux profils de déplacement, déplace une aiguille d'injection insérée (4) par rapport à une ampoule insérée (3) et, dans l'autre des deux profils de déplacement, déplace une aiguille d'injection insérée (4) conjointement avec une ampoule insérée (3).

12. Appareil d'injection selon l'une quelconque des revendications précédentes, **caractérisé par** un capteur (40, 41, 42) pour enregistrer un réglage de l'appareil effectué de manière mécanique, en particulier en ce qui concerne la profondeur de piqûre réglée ou bien la quantité d'insuline à injecter.
